# EUROPEAN PATENT SPECIFICATION

(11) **EP 2 317 452 B1**
(45) Date of publication and mention of the grant of the patent: **13.04.2016**
(21) Application number: 10188935.0
(22) Date of filing: 26.10.2010
(51) Int. Cl.: G06F 19/00, A61B 5/1455

(54) **Multiple wavelength physiological measuring apparatus, sensor and interface unit for determination of blood parameters**
Physiologisches Messgerät mit mehreren Wellenlängen, Sensor und Schnittstelleneinheit zur Bestimmung von Blutparametern
Appareil de mesure physiologique de longueur d'onde multiple, capteur et unité d'interface pour la détermination des paramètres du sang

(30) Priority: 28.10.2009 US 607172
(43) Date of publication of application: 04.05.2011
(73) Proprietor: GENERAL ELECTRIC COMPANY, Schenectady, NY 12345 (US)
(72) Inventor: Huiku, Matti, 02760, Espoo (FI)
(74) Representative: Illingworth-Law, William Illingworth

(56) References cited:
- WO-A1-01/52727
- WO-A2-2007/143626
- US-A1- 2008 183 232

## Description

### Background of the Invention

This disclosure relates to multiple optical wavelength physiological sensors and monitors, especially to pulse oximeters.

Pulse oximetry is a well-established technique for measuring oxygen saturation (SpO₂) in arterial blood. SpO₂ is an important parameter that relates to the adequacy of oxygen supply to peripheral tissues and organs. Pulse oximeters provide instantaneous in-vivo measurements of arterial oxygenation, and thereby an early warning of arterial hypoxemia, for example. Pulse oximeters also display a photoplethysmographic (PPG) pulse waveform, which can be related to tissue blood volume and blood flow, i.e. the blood circulation, at the site of the measurement, typically in finger or ear.

Since the measurement is normally made from an anatomical extremity, such as a finger tip, pulse oximeters typically comprise a separate sensor, attachable to a subject, and the actual pulse oximeter device to which the sensor is connected through a cable. Standard pulse oximeters use two wavelengths to measure the ratio of oxyhemoglobin to total functional hemoglobin, indicated as an SpO₂ value. The sensor of a standard pulse oximeter therefore comprises two emitter elements, each emitting radiation at a specific wavelength, and a photodetector common to the emitter elements. Although standard pulse oximeters require only two wavelengths, multiwavelength pulse oximeters provided with more than two wavelengths are becoming more and more common, since they provide higher performance and wider applicability. For example, levels of other significant hemoglobin species, such as carboxyhemoglobin and methemoglobin and total hemoglobin, may be estimated if the number of wavelengths used in the pulse oximeter is increased. The sensor of a multiwavelength pulse oximeter therefore comprises more than two, typically 6 to 12, emitter elements, and a broad spectral band photodetector common to all emitter elements.
WO 2007/143626 discloses a physiological monitor having a sensor port configured to attach and communicate with a sensor. Upgrade tools are individually attachable to the sensor port in lieu of the sensor to designate to the processor board which of the physiological parameters to calculate when the sensor is attached to the sensor port.

One drawback of the current oximeters is that they do not inform the user about possible accuracy issues the user may encounter while using a sensor that has degraded in performance. The accuracy of a measurement normally depends on a plurality of variables, such as the center wavelengths of the emitter elements, which drift in the course of time, resulting in degradation of the sensor performance. The accuracy issue is also related to the measurement in question and to the current trend towards an increasing number of wavelengths. This trend means that the measurements become more complex and thus also more sensitive to the changes occurring in the sensor over time and as a result of the use of the sensor.

As the pulse oximeters cannot analyze the performance level of the sensor, the problem is at present tackled typically so that the sensor manufacturer sets an upper limit for the usage time of the sensor, after which the sensor is to be replaced by a new sensor. However, this is not the best possible solution for the problem, since the sensor degrade rate depends on the operating conditions and since all wavelengths may not be used similarly and may thus not be subject to similar degradation in the course of time. In addition, the vulnerability of different measurements to sensor degradation may vary, due to the different accuracy requirements of the measurements. The setting of an upper limit for the usage time of the sensor thus easily leads to waste of resources, since the upper limit is to be set with a safety margin.

Another drawback of the current pulse oximeters is that the sensors must be used as they are originally configured at the manufacturer. A multiwavelength sensor may, however, intrinsically support many other measurement options than those originally configured for the sensor. However, such measurement options cannot be taken into use during the lifetime of the sensor, since the use of the sensor is limited to the original configuration carried out at the stage of manufacture.

### Brief Description of the Invention

The above-mentioned problems are addressed herein which will be comprehended from the following specification.

The present invention provides a measuring apparatus as defined in claim 1 and an interface unit as defined in claim 11.

Various other features, objects, and advantages of the invention will be made apparent to those skilled in the art from the following detailed description and accompanying drawings.

### Brief Description of the Drawings

FIG. 1 is a block diagram illustrating the basic configuration of a conventional pulse oximeter;
FIG. 2 illustrates an embodiment of a multiwavelength pulse oximeter;
FIG. 3 illustrates the drive pulse sequences of two measurement modes of the multiwavelength pulse oximeter of FIG. 2;
FIG. 4 illustrates an example of the emitter switching unit and emitter driver unit of the embodiment of FIG. 2;
FIG. 5 illustrates one embodiment of the emitter unit and the emitter switching unit of the pulse oximeter of FIG. 2;
FIG. 6 is a flow diagram illustrating an example of the operation of the sensor ability maintenance unit;
FIG. 7 is a flow diagram illustrating an example of the update of the diagnostic data and service call index; and
FIG. 8 illustrates a further embodiment of the emitter unit of the pulse oximeter of FIG. 2.

### Detailed Description of the Invention

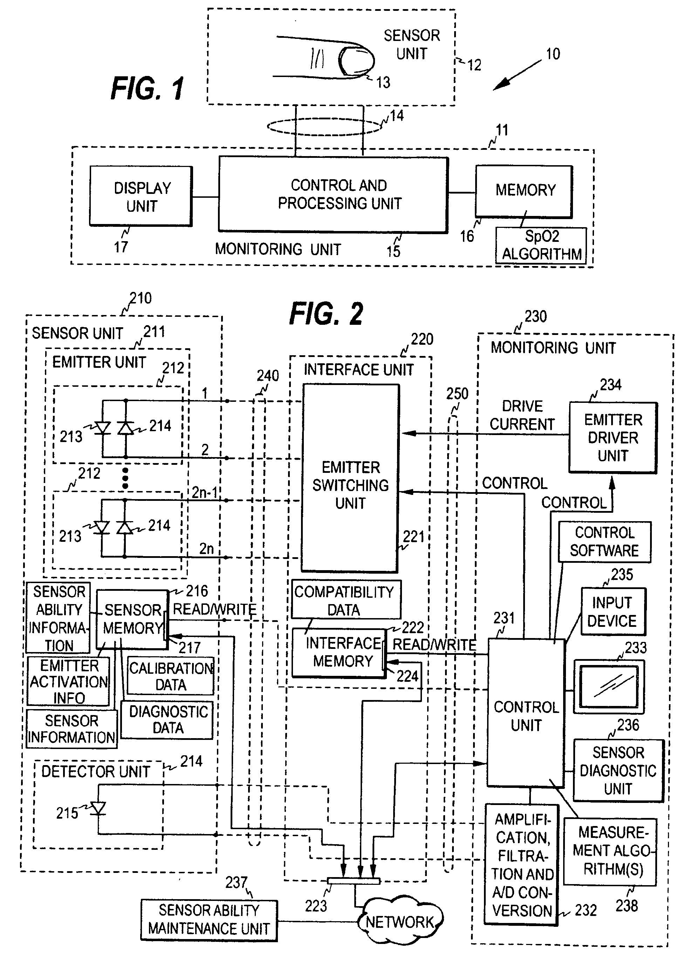

FIG. 1 illustrates the basic elements of a conventional pulse oximeter 10. A pulse oximeter normally comprises a bedside monitoring unit 11 and a probe or sensor unit 12 attachable to a subject, typically to a finger 13 or ear lobe of the subject. The sensor unit is normally connected to the monitoring unit through a cable 14. The monitoring unit may be conceived to comprise three basic elements: a computerized control and processing unit 15, a memory 16 for the control and processing unit, and a display 17 for displaying information to a user of the pulse oximeter.

The sensor unit normally includes light sources for sending optical signals through the tissue and a photodetector for receiving the signals transmitted through or reflected from the tissue. On the basis of the transmitted and received signals, light absorption by the tissue may be determined. During each cardiac cycle, light absorption by the tissue varies cyclically. During the diastolic phase, absorption is caused by venous blood, non-pulsating arterial blood, cells and fluids in tissue, bone, and pigments, whereas during the systolic phase there is an increase in absorption, which is caused by the inflow of arterial blood into the tissue part on which the sensor is attached. Pulse oximeters focus the measurement on this pulsating arterial blood portion by determining the difference between the peak absorption during the systolic phase and the background absorption during the diastolic phase. Pulse oximetry is thus based on the assumption that the pulsating component of the absorption is due to arterial blood only.

In order to distinguish between two species of hemoglobin, oxyhemoglobin (HbO₂) and deoxyhemoglobin (RHb), absorption must be measured at two different wavelengths, i.e. the sensor of a traditional pulse oximeter includes two different light emitting diodes (LEDs) or lasers. The wavelength values widely used are 660 nm (red) and 940 nm (infrared), since the said two species of hemoglobin have substantially different absorption at these wavelengths. Each LED is illuminated in turn at a frequency which is typically several hundred Hz. If the concentrations of more than said two hemoglobin species are to be evaluated, more than two wavelengths are needed. Such a pulse oximeter is here termed a multiwavelength pulse oximeter.

The light propagated through or reflected from the tissue is received by a photodetector, which converts the optical signal received at each wavelength into an electrical signal pulse train and feeds it to an input amplifier. The amplified signal is then supplied to the control and processing unit 15, which converts the signals into digitized format for each wavelength channel. The digitized signal data is then utilized by an SpO₂ algorithm. The control and processing unit executes the algorithm and drives the display 17 to present the results on the screen thereof. The SpO₂ algorithm may be stored in the memory 16 of the control and processing unit. The digitized photoplethysmographic (PPG) signal data at each wavelength may also be stored in the said memory before being supplied to the SpO₂ algorithm. With each LED being illuminated at the above-mentioned high rate as compared to the pulse rate of the subject, the control and processing unit obtains a high number of samples at each wavelength for each cardiac cycle of the subject. The time windows corresponding to a particular wavelength are often referred to as a wavelength channel.

FIG. 2 illustrates one embodiment of a multiwavelength pulse oximeter. The sensor unit 210 is in this case connected to the monitoring unit 230 through an interface unit 220 which in this example includes an emitter switching unit 221 and a memory 222. The interface unit 220 is in this example a separate module connected to the monitoring unit 230 through a connector 250. However, the interface unit may also be integrated with the monitoring unit 230 or with the cable 14. The element 240 connecting the interface unit to the sensor unit may thus comprise a connector and/or a cable. As discussed below, the interface unit serves to facilitate a use of different types of sensors without making the actual monitoring unit too complex. The interface unit also facilitates a modular multiwavelength design, in which the monitoring unit 230 may include only essential signal processing for the different possible signal trains and one electric current source unit that can serve multiple light sources. The interface unit may or may not be provided with a dedicated memory 222, regardless of whether the unit is a separate module or integrated with the monitoring unit or the cable. In FIG. 2, the access interface of the memory of the interface unit is denoted with reference number 224.

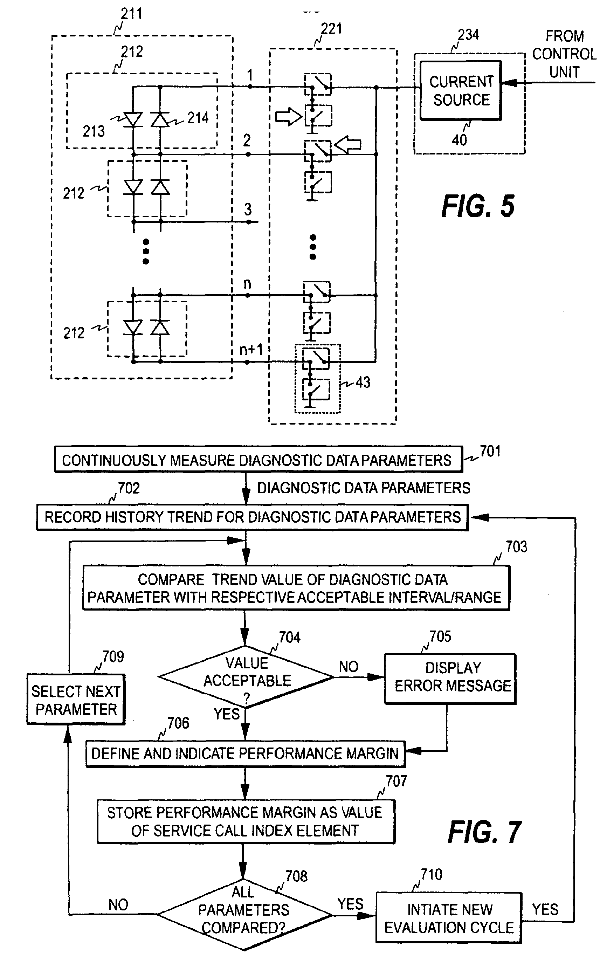

The sensor unit 210 of FIG. 2 comprises an emitter unit 211 comprising n (n≥2) emitter element units 212 each comprising two emitter elements (LEDs or lasers) 213, 214 connected in parallel and back-to-back, i.e. in each emitter element unit the anode of the first emitter element and the cathode of the second emitter element are connected together and form a first common pole, while the cathode of the first emitter element and the anode of the second emitter element are connected together to form a second common pole. The said poles form the terminals of one emitter element unit 212, while the terminals of all emitter element units form the terminals of the emitter unit. As illustrated in the figure, the total number of the said terminals is 2n in this embodiment. Each emitter element may be adapted to emit radiation at a dedicated wavelength, i.e. the number of wavelengths may also be 2n. However, the number of wavelengths may also be lower, if all units 212 do not include two emitter elements or if two or more units 212 comprise substantially the same wavelengths. Furthermore, as discussed below in connection with FIG. 5, the n emitter element units may also be cascaded. In this kind of arrangement, the total number of terminals is n+1, but the number of wavelengths may still be 2n, if all emitter element units include two emitter elements and all emitter elements have different wavelengths.

The sensor unit 210 further comprises a sensor memory 216 and a detector unit 214 comprising a broad spectral band photodetector 215 adapted to receive the radiation emitted by the emitter elements and to convert the optical signals into electric signals.

In the monitoring unit 230, the control and processing unit and the associated memory is illustrated as a control unit 231. In addition to the above basic elements, the monitoring unit of FIG. 2 comprises a reception branch 232 adapted to receive the electric signals from the photodetector and an emitter driver unit 234 adapted to generate, under the control of the control unit, drive current for the emitter elements. The reception branch 232 typically comprises an input amplifier, a band-pass filter, and an A/D converter (not shown). The digitized signal output from the A/D converter is supplied to the control unit 231, which processes the signal data and displays the analysis results on the screen of a display unit 233. The control unit is provided with control software for controlling the activation of the emitter elements in the emitter element units by controlling the emitter driver unit 234 and the emitter switching unit 221 in a synchronized manner. Therefore, the control unit also knows from which one of the emitter elements the signal data originates in each time window. The drive current generated in the emitter driver unit is supplied to the emitter switching unit 221. The control unit controls the switches of the emitter switching unit so that a repeating drive pulse sequence is generated, each pulse thereof being supplied to the correct emitter element (i.e. LED or laser). The required control information may be produced based on the emitter activation information stored in sensor memory 216.

For addressing the above-mentioned problems of current multiwavelength pulse oximeters, the sensor memory 216 may store various sensor-specific information about the sensor unit and the memory is provided with an access interface 217 for enabling an entity external to the sensor unit to update at least part of the sensor-specific information. In the embodiment discussed below, the sensor-specific information may be divided into five data sets: sensor information, calibration data, emitter activation information, sensor ability information, and diagnostic data. Below, the five types of data sets are discussed in more detail.

The sensor information includes sensor-specific identification data, such as the type (finger/ear/adult/infant/neonatal, etc.), the specified use (total hemoglobin, carboxyhemoglobin, methemoglobin or standard SpO₂ measurement) and the identifier of the sensor in question. The identifier may be, for example, the serial number of the sensor.

The calibration data may include various data that the measurement algorithms stored in the control unit may utilize. For example, the calibration data may include the following data: extinction coefficient data, center wavelengths used in the sensor, temperature coefficients for wavelength temperature shift, nominal tissue parameters at calibration conditions, and sensor optics and design characteristics, such as sensor nominal current transfer ratios. The extinction coefficient data includes the extinction coefficients related to each wavelength/blood substance pair, i.e. each extinction coefficient indicates the absorption of the said blood substance at the wavelength in question. The temperature coefficients indicate how the center wavelengths change as a function of temperature and the tissue parameters indicate, for instance, how the transmission in the tissue affects the spectral characteristics seen by the detector, i.e. how the tissue shifts the center wavelength. The current transfer ratios (CTRs) indicate the ratio of the detector output current to the LED input current for each LED/detector pair while there is no tissue between the detector and the LEDs.

The emitter activation information stored in the sensor memory includes information indicating how the emitter unit is to be driven to generate an optical signal at a desired wavelength. The said information may be combined with the extinction coefficient data, for example. The combined information may be in the form of a table, as is shown in Table 1 below.

**Table 1.**

| Terminals | Current polarity | RHb | HbO2 | HbCO | HbMet | HbX | Center Wavel. (nm) |
|---|---|---|---|---|---|---|---|
| 1, 2 | Plus | ε_{RHb,632} | ε_{HbO2,632} | ε_{HbCO,632} | ε_{HbO2,632} | ε_{HbX,632} | 632 |
| 1,2 | Minus | ε_{RHb,660} | ε_{HbO2,660} | ε_{HbCO,660} | ε_{HbO2,660} | ε_{HbX,660} | 660 |
| 3,4 | ··· | ··· | ··· | ··· | ··· | ··· | ··· |
| ··· | ··· | ··· | ··· | ··· | ··· | ··· | ··· |
| 2n-1, 2n | Minus | ε_{RHb,940} | ε_{HbO2,940} | ε_{HbCO,940} | ε_{HbO2,940} | ε_{HbX,940} | 940 |

In table 1, the first, second and last columns indicate how the emitter unit is to be driven to generate a signal at a specific wavelength. The first row of the said columns indicates that if a drive current is supplied from terminal 1 to terminal 2 (i.e. positive current with respect to terminals 1 and 2), a signal having a center wavelength of 632 nm is generated. Similarly, the second row of the said columns indicates that if a drive current is supplied in the opposite direction between terminals 1 and 2 of the emitter unit (i.e. negative current with respect to terminals 1 and 2), an optical signal having a center wavelength of 660 nm is generated. Columns 3-7 of table 1 include, respectively, the extinction coefficients of deoxyhemoglobin (RHb), oxyhemoglobin (HbO₂), carboxyhemoglobin (HbCO), methemoglobin (metHb), and one further hemoglobin species (HbX) at the wavelengths used in the emitter unit. It is assumed in table 1 that the shortest wavelength is 632 nm and the longest 940 nm, and that the emitter unit is provided with 2n drive input terminals.

The emitter activation information in the sensor memory may also be in the form of control codes, for example, in which case the control unit may map each code to the control information needed to employ a particular set of wavelengths.

The sensor memory further includes the above-mentioned sensor ability information. This information indicates whether a predetermined sensor ability update process needs to be initiated to extend the usage of the sensor. The update process is typically initiated before the sensor unit is to be used in a certain new measurement mode that the user wishes to employ. In some embodiments, an ongoing measurement is not stopped due to a detected need to perform a sensor ability update process, but the user is only informed of the need. However, in some other embodiments the ability update process may also be started during the measurement, if it is detected that the performance of the sensor has degraded below an acceptable level, thereby to extend the use time of the sensor in the current measurement mode. A measurement mode here refers to a certain type of measurement that utilizes a dedicated set of wavelengths. However, measurement modes using the same combination of wavelengths but different combination of emitter elements are in this context considered as different measurement modes. The sensor ability update process prepares the sensor unit for the measurement mode to be initiated by updating the ability of the sensor unit. As discussed below, the sensor ability information may also be stored in the interface unit. In one embodiment, the content of the sensor ability information may be divided into two categories; activation status information indicating whether or not the sensor unit needs to be reconfigured before the selected measurement mode can be initiated and service information indicating whether the sensor unit needs to be serviced or recalibrated for the measurement mode selected or in progress. If the activation status information indicates that the sensor is currently configured to operate in the said measurement mode, the sensor is in principle ready to be used in the selected measurement mode. However, the performance of the sensor may at present be degraded below an acceptable level with respect to the measurement mode to be initiated or in progress. The service information indicates if this is the case.

The control unit 231 is further provided with a sensor diagnostic unit 236 configured to collect various history data regarding sensor usage and drift characteristics. Although the functionalities carried out by the sensor diagnostic unit may be implemented within the control unit, the sensor diagnostic unit is presented here as a separate functional entity. As discussed below, the history data collected by the sensor diagnostic unit is employed to maintain/update the data in the sensor memory 216. The purpose of the history data collection is to enable tracking of the possible degradation of the sensor and the detection of certain failure modes of the sensor before the accuracy of the measurement is affected. The diagnostic data is in this example stored in the sensor to make the said data available when the sensor is used in another monitoring unit.

The diagnostic data stored in the sensor memory typically includes the history data recorded by the sensor diagnostic unit 236 in response to the use of the sensor unit in the pulse oximeter. The diagnostic data may include, for example, the number of hours that the sensor has been used in the device and temperature records including operating temperatures measured during the use of the sensor. A high operating temperature can cause subject's skin tissue necrosis or a burn. The diagnostic data can thus also reveal a potentially hazardous situation and disable the use of the sensor by updating the sensor ability information correspondingly. Another typical failure mechanism of a sensor is that the emitter intensity decreases during the lifetime of the sensor. In order to track the degradation of the sensor, the diagnostic data may include a history record of the current transfer ratios (CTRs) that can be measured when the sensor is off the finger or ear. A low value of CTR indicates that the emission intensity in the particular wavelength channel is inadequate for the measurement. Further, the wavelengths in the sensor may shift due to high temperature, but also due to the degradation of the emitter components. In the worst case, the measurement accuracy is compromised in certain measurement modes. The measurement algorithm 238 in the monitoring unit 230 may calculate a quality index or a residual error index that indicates a poor convergence of the algorithm. A continuation of the poor convergence case by case may be an indication of sensor degradation. The residual error may thus be one of the diagnostic data parameters for which history data is recorded. Persisting high values of the residual error may initiate a sensor ability update process, during which the sensor calibration data is updated for maintaining high accuracy in all measurement modes. Another way to detect wavelength shifts is presented in U.S. Patent 6,501,974. This patent suggests that a s.c. pseudo-isobestic invariant be calculated from at least 3 wavelength signals. The wavelength shift can now be detected, if, for instance, two of the wavelengths belong to the actual measurement set and are normally activated in each emitter activation cycle, but one wavelength is reserved for occasional use to check the value of the pseudo-isobestic-invariant (PII). If PII changes from the nominal value, a wavelength shift in the two other wavelengths may have occurred. Pseudo-isobestic invariants specific to certain wavelength combinations may thus be included in the diagnostic data parameters for which history data is recorded. In one embodiment, the history data is in the form of parameter-specific history trends. That is, the diagnostic data stored in the sensor memory includes a history trend for each diagnostic data parameter. The required storage capacity may be reduced if history trends are stored instead of plain history data. The length of the stored history trend may vary, but would preferably be at least one data point per one patient monitoring case or one data point per hour, for example. Each trend point may represent an average of the trended parameter values over the above-mentioned trend interval. For clarity reasons, the various elements possible for measuring the data for the diagnostic data parameters are not shown in FIG. 2.

The sensor ability information thus includes information that indicates whether a predetermined sensor ability update process, such as sensor reconfiguration and/or recalibration, needs to be initiated before the sensor unit can be used in the desired measurement mode or before an ongoing measurement can be continued. In the embodiment disclosed below, the sensor ability information is divided into two categories; activation status information and service information. The activation status information may include a list of capabilities for which the sensor unit is at present configured, and possibly also a list of capabilities/resources that are currently inactive but which may be activated, if needed, by reconfiguring the sensor unit. As discussed below, the sensor unit may be configured for a wide variety of measurement modes. However, some of the resources available in the sensor may be preserved for future use and may thus be inactive at present. The activation status information indicates if some of the resources available in the sensor need to be activated before a certain measurement mode can be initiated. The activation status information may also be combined with the emitter activation information. That is, the content of the emitter activation information, such as the terminal information revealed in the first and second columns of table 1, may indicate the activated resources, whereas the lack of the same information with respect to one or more wavelengths may indicate the inactivated resources (wavelengths). In logic sense, the activation status information may thus be stored as explicit data or as lack of certain data, such as lack of emitter activation information of wavelengths that remain inactivated. It is therefore to be noted that the activation status information may take various logical forms.

The service information may include a service call index that indicates whether the sensor needs to be recalibrated or serviced before a certain measurement mode can be initiated. As discussed below, the service call index may comprise a plurality of index elements that may, independently or in combination, be indicative of a certain cause of a performance drop.

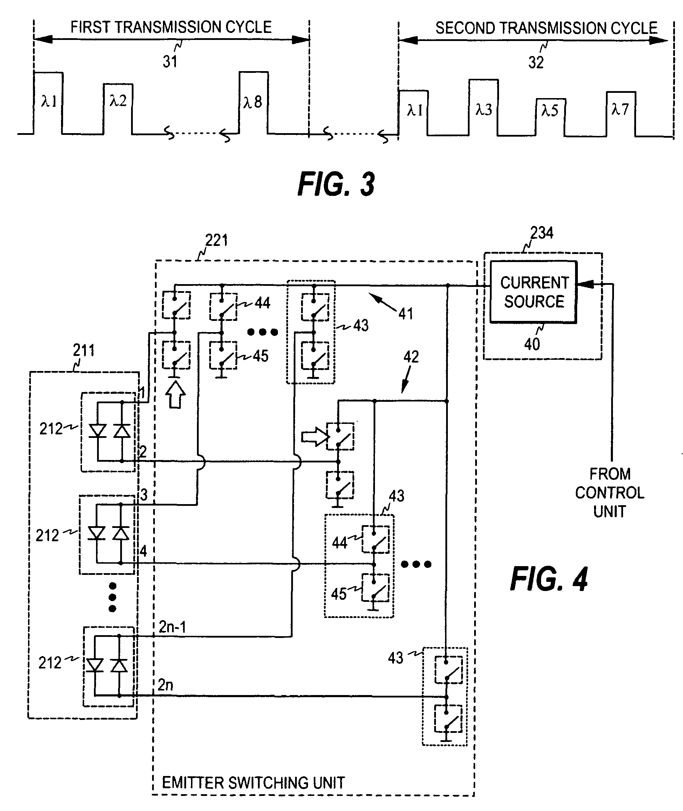

The control unit 231 may read the content of sensor memory 216 through the interface unit, thereby to determine the operations needed before the control information can be generated to activate the required LEDs only. FIG. 3 illustrates the repeating drive pulse sequences 31, 32 for two measurements. In the first measurement, 8 wavelengths are needed for the measurement, which are in this example wavelengths λ1- λ8. The control unit therefore produces control information that controls the emitter driver unit 234 and the emitter switching unit 221 so that the LEDs corresponding to wavelengths λ1- λ8 are activated in desired order using an appropriate drive current for each LED. In the second measurement, four wavelengths are needed, which are in this example wavelengths λ1, λ3, λ5, and λ7. Again, the control unit may determine, based on the emitter activation information, the control information needed to activate the required LEDs only, as is shown in FIG. 3. Thus, in FIG. 3 the wavelength marks within each drive pulse indicate that at that time slot the control information supplied by the control unit to the emitter switching unit 221 is such that only the LED corresponding to that wavelength is activated. Consequently, the number of pulses in each repeating pulse sequence corresponds to the number of wavelengths needed. However, as mentioned above, the wavelengths may be activated in a desired order within the pulse sequence.

The above LED control modes, which activate the required LEDs only, enable optimal time division multiplexing of the wavelength channels that are needed for the measurement. Furthermore, as the combination of wavelengths may be selected flexibly using the emitter activation information, the combination of the wavelengths employed may be changed dynamically over time. For example, the dynamical alternation of the combination may depend on the blood parameters to be tracked and on the rate at which the said parameters may change; parameters that may change faster may be measured more frequently than parameters having a slower rate of change. The above features improve the signal-to-noise ratio and, thereby, accuracy of the particular measurement. The above control modes may also be sensor-specific: the control unit may use one or more LED control modes for one sensor type and one or more other control modes for another sensor type. In this case the emitter activation information in the sensor memory 216 may comprise the control codes for the control modes compatible with the sensor. The control unit may retrieve the control code corresponding to the wavelength combination to be employed and use the retrieved control code to produce the control information for the corresponding LEDs. The emitter activation information may thus include the required information for each emitter element separately, as in table 1, or for each wavelength combination possible with the sensor. The information may also be in the form of access codes that the control unit may use to retrieve the required information from another location, such as from a local memory.

FIG. 4 illustrates one embodiment of the emitter driver unit 234 and the emitter switching unit 221 of FIG. 2. For reasons of clarity, other elements except emitter unit 211 have been omitted in the figure. The emitter current source comprises in this example a single current source 40, which outputs the drive current for the pulse sequences of FIG. 3. In this embodiment, drive current for the first emitter elements (LEDs) in all emitter element units 212 is supplied through output branch 41, while the drive current for the second emitter elements (LEDs) in all emitter element units is supplied through output branch 42. In other words, current source 40 is connected to the anodes of the first emitter elements (LEDs) in the emitter element units through output branch 41, and to the anodes of the second emitter elements (LEDs) of the emitter element units through output branch 42. The connection is formed through the emitter switching unit, which comprises n switching units 43 in each output branch. Each of the 2n switching units 43 comprises a first switching element 44 and a second switching element 45 connected in series. If, for example, the emitter activation information indicates that wavelength λ2 is to be produced by supplying current from terminal 2 to terminal 1 of the emitter unit, the control unit generates, in the time slot corresponding to wavelength λ2, a drive pulse amplitude suitable for the corresponding LED and closes the switching elements indicated by the arrows in the figure, while leaving other switching elements open. The number of the switching units used in the emitter switching unit may correspond to the maximum number of sensor drive terminals (i.e. input terminals of the emitter unit), thereby to make the emitter switching unit compatible with all possible sensors.

FIG. 5 illustrates another embodiment of the emitter unit 211 and the emitter switching unit 221 of the pulse oximeter of FIG. 2. In this case the emitter element units 212 are cascaded, i.e. the second common pole in an emitter element unit is connected to the first common pole in the next emitter element unit. Although there are still n emitter element units 212 in the embodiment of FIG. 2, the number of the input terminals of the emitter unit is now reduced to n+1. The same applies to switching units 43, i.e. the number of the output terminals of the interface unit is also n+1. The arrows indicate the two switching elements to be closed when the same LED as in the example of FIG. 4 is to be activated.

When the apparatus comprises the interface unit provided with a dedicated memory, at least some of the above information of the sensor memory may be stored in the interface memory only. Furthermore, the sensor-specific information, which in the sensor memory concerns that sensor only, may in the interface memory concern a plurality of different sensors that have been used with the interface unit or that are intended to be used with the interface unit. For example, the diagnostic data may be collected into the memory of the interface unit for all sensor IDs that have been used with the interface unit. The interface memory may also contain additional information, such as general compatibility data indicating the sensor types that are compatible with each possible measurement mode (wavelength set).

As discussed above, the emitter activation information indicates how the emitter unit is to be driven to generate an optical signal at a desired wavelength. However, if the emitter activation information is stored in the interface unit, it may include the activation information or the control codes needed for all measurement modes (i.e. for all LED control modes) possible with a plurality of monitoring units with different measurement capabilities. Each measurement mode corresponds to a specific wavelength set and the emitter activation information may include the switching control data and the drive pulse data for each set. In this way, each control unit does not have to determine the above-described control information, but may simply retrieve the said information or the control code from the interface unit memory for the wavelength combination to be employed. Furthermore, monitoring units with different measurement capabilities may utilize the same or the same type of interface unit, since they can all read the emitter activation information corresponding to their wavelength set(s). When the interface unit stores the emitter activation information for several measurement modes, the control unit may read the sensor information in the sensor memory to ascertain that the sensor is compatible with the measurement mode.

At logical level, the control unit 231 and the associated diagnostic unit 236 form a functional entity that updates the sensor-specific information in the sensor memory so that the sensor ability may be updated and/or maintained for the measurement mode in question. In the embodiment discussed below, this entity may further inform the user about the possible need to update the sensor ability for the measurement mode that the user wishes to initiate or is currently using, i.e. about the need to perform the sensor ability update process. This functional entity is here termed sensor ability maintenance unit. The entity may also inform the user about compatibility issues, if general compatibility data is stored in the apparatus.

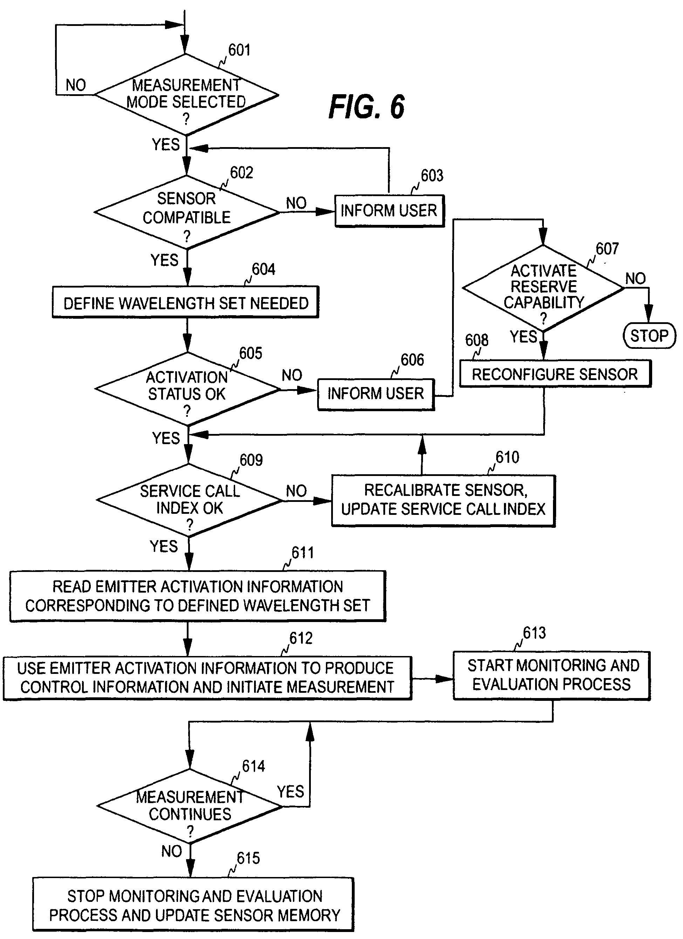

FIG. 6 is a flow diagram illustrating an example of the operation of the control and diagnostic units. When the user of the device has chosen a certain measurement mode through the user interface of the device (step 601), the control unit reads the sensor type information and the general compatibility data to determine whether a compatible sensor is connected to the device (step 602). If this not the case, the user is informed to change the sensor. This may involve displaying the sensors compatible with the selected measurement mode (step 603). The control unit may store a compatibility guide that the user may use when operating the device. When a compatible sensor is connected to the monitoring unit, the control unit determines the wavelength set to be employed (step 604) and reads the activation status information to determine whether the resources necessary for the measurement mode have been activated (step 605). If some of the resources needed for the measurement have not been activated, the user may be informed of the need to activate some of the resources available in the sensor (step 606). If the user accepts the activation (step 607/yes), the control unit reconfigures the sensor for the selected measurement mode (step 608). In addition to the update of the activation status information, this may also involve the update of the emitter activation information stored in the sensor memory. As an example of the reconfiguration process, new sensor current terminals may be taken in use in order to facilitate new wavelength channels for the new active measurement. This may be carried out, for example, by writing the terminal numbers and the polarity information into table 1.

After the above steps, the control unit examines the service information stored in the sensor to check whether recalibration or service is needed (step 609). If the current value of the service call index indicates that recalibration of the sensor unit is needed, the control unit retrieves new calibration data, uses the said data to recalibrate the sensor, and updates the service call index (step 610). Recalibration here refers to the update of the calibration data and to the other operations that are possibly needed to provide new parameter values for the measurement algorithm so that the sensor becomes "recalibrated" form the point of view of the measurement algorithm. A need for recalibration typically arises from the gradual degradation of the sensor components. An example of the recalibration process may be a situation in which the sensor ability maintenance unit, by analysis of the diagnostic data, detects a wavelength shift in one or two emitter components. The service call index simultaneously indicates that the more advanced measurement modes, like the one selected above, are compromised due to the potentially poor accuracy. The sensor ability maintenance unit may now inform the user about the situation by displaying an error code/message at step 610. User acceptance for the recalibration may be prompted in step 610 and the error code/message may also suggest that the sensor wavelengths should be measured and new wavelength values or full emission spectra should be given to the system. After the user has measured the sensor wavelengths, (s)he may input the new center wavelength values through the input device 235. The measurement may be made by an external spectrometer device, for example. Alternatively, the full measured emission spectra may be analyzed in an internet network service that may then calculate new extinction coefficients for the sensor and return the new values to the apparatus. Consequently, step 610 may involve user interaction for measuring the center wavelengths of the sensor unit. After the recalibration, the service call index may be reset, in step 610, to a value indicating full or partial performance in the system. The new calibration data may also be retrieved from a local memory.

To enable connections with external network elements, the interface or monitoring unit of FIG. 2 may be provided with a network port 223 through which the memories of the pulse oximeter may be updated. Thus, it is also possible that the update of the sensor memory data and/or the interface memory data is carried out, upon request from the control unit, by an external device through the network port. Consequently, at least some functionalities of the sensor ability maintenance unit may be in the network, as is denoted with an external sensor ability maintenance unit 237 in FIG. 2. To retrieve the new calibration data, the control unit may form a data set including the current service call index, the identifier of the sensor, and a diagnostic profile formed based on the diagnostic data. This set is then used to find and retrieve the correct calibration information for the sensor unit.

If the service call index value indicates at step 609 that the performance of the sensor is acceptable for the selected measurement mode, the control unit reads the emitter activation information that corresponds to the combination of wavelengths to be employed (step 611), produces the control information based on the information read (step 612), and initiates the actual measurement. Upon initiation of the measurement, the sensor ability maintenance unit starts a background monitoring and evaluation process 613, in which the diagnostic data and the service call index are updated according to the actual use of the apparatus (step 613). This process runs during the actual measurement. When the actual measurement is stopped and the sensor is removed from the measurement site (step 614/yes), the monitoring and evaluation process is also stopped and the sensor memory is updated for the next measurement at step 615. This may involve performing one evaluation cycle shown in FIG. 7, thereby to update the sensor memory (service information) to correspond to the situation at the end of the actual measurement.

In the above embodiment, each of the check steps (602, 605 and 609) and step 611 involves the reading of the required data from the sensor memory. However, the content of the sensor memory data may also be read at a go before the actual measurement starts while the sensor is plugged in the monitor (i.e. before or after step 601). Steps 601-615 are then executed as discussed above, but without reading each type of data separately from the sensor memory.

In one embodiment, the service call index may be an array of index elements, in which each index element is indicative of a certain sensor failure mechanism that is associated with certain diagnostic data parameter(s). For instance, the wavelength shift error can be detected based on the residual error parameter and/or on values of certain pseudo-isobestic invariants, as described above. The diagnostic CTR values of each wavelength channel can be associated with degrading emitter intensity, while an increased sensor temperature at a fixed channel drive current may indicate that the sensor is mechanically damaged. A typical degradation of sensor performance is due to a dirty sensor that associates with low CTR and poor residual error, i.e. a combination of two diagnostic data parameters.

Figure 7 illustrates an example of the monitoring and evaluation process of step 613, assuming that the service call index is an array of index elements, each index element being derived from a respective diagnostic data parameter. As discussed above, the monitoring and evaluation process may be a background process running during the actual measurement. The diagnostic data parameters, such as wavelength-specific CTRs, residual error, and pseudo-isobestic invariants, are substantially continuously derived from the diagnostic data at step 701. However, in order to reduce the amount of data to be stored in the sensor, the sensor diagnostic unit determines history trends for the diagnostic data parameters at certain time intervals and stores the history trends in the sensor memory (step 702). Based on the history trend, a trend value of each diagnostic data parameter is then determined and compared with respective acceptable range/interval at step 703. The determined trend value may be the current trend value indicated by the stored history trend or a subsequent trend value that is expected after a short period of time, such as 5 minutes. The acceptable range/interval of each diagnostic data parameter depends on the measurement mode; the more demanding the measurement, the tighter the acceptable range/interval. If the parameter value is not within the acceptable range, an error code/message is displayed that informs the user about the reason of the error (step 705). The sensor ability maintenance unit then determines a performance margin for the parameter and indicates the value thereof to the user (step 706). The performance margin may be defined as the percentage of remaining distance to/from a predetermined threshold value (i.e. end point of the acceptable range). The performance margin is then stored as the current value of the respective service call index element (step 707). Steps 703 to 707 are performed for each diagnostic data parameter, thereby to obtain each service call index element. Consequently, the actual measurement is not stopped in step 705, but a message is displayed to the user if the diagnostic data parameter is not within the acceptable range. In this case the resulting performance margin indicated in step 706 is negative. When a check carried out in step 708 indicates that all parameters have been compared with the respective acceptable range, the start of a new evaluation cycle is determined in step 710 and the new evaluation cycle is started. The determination typically involves setting a timer. When the timer expires, the new evaluation cycle is started and the process jumps from step 710 to step 702 to record the history trends based on the newest values of diagnostic data parameters and the newest trend values are again compared with the respective acceptable ranges. If the service call index comprises a plurality of index elements, each index element is examined in step 609 for the measurement mode in question to check whether any of the possible failure modes has caused a drop in the performance of the sensor unit.

In the above manner the history trends and the index element values stored in the sensor memory may be updated at regular intervals, such as every 20 or 30 minutes, and also at the end of the measurement. That is, step 615 may include steps 702-709 to update the service information to correspond to the situation at the end of the actual measurement.

Depending on the nature of the diagnostic data parameter, the parameter measurement and recording of history trends may be carried out when the sensor is not in operation (cf. CTR). The evaluation cycle is therefore not necessarily performed for all diagnostic data parameters during the actual measurement, but for one or more diagnostic data parameters the above update of the respective index element(s) may be carried out only at the end of the actual measurement.

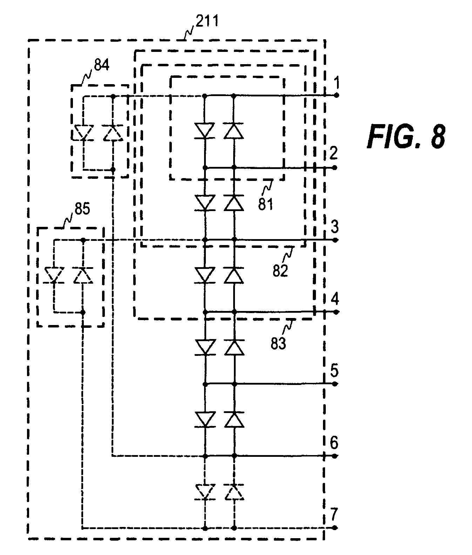

FIG. 8 illustrates a further example of the emitter unit of the pulse oximeter of FIG. 2. The emitter unit comprises in this case 8 emitter element units each comprising two emitter elements (LEDs or lasers) connected in parallel and back-to-back, i.e. the total number of the emitter elements is 16. However, in this case the number of the drive input terminals of the sensor unit is only 7, since two of the emitter element units, denoted with reference numbers 84 and 85, have been added in parallel with a cascade of five and four emitter element units, respectively. Additional LEDs may be added in parallel with a cascade of at least 3 or 4 LEDs, thereby to decrease the number of input terminals required in the sensor unit. However, to ensure that the drive current of such an additional LED will not leak through the cascaded LEDs connected between the same input terminals, the voltage over the activated additional LED must be less than the sum of the opening threshold voltages of the said cascaded LEDs. Therefore, the number of said cascaded LEDs must in practice be at least 3 or 4. A sensor unit comprising 2n emitter elements may thus also include less than n+1 drive input terminals. This also decreases the number of switching units 43 and the number of output terminals in the interface unit, as is obvious from FIGs. 4 and 5.

A sensor unit provided with the emitter unit of FIG. 8 may be manufactured with a full capacity of 16 emitter elements. However, the sensor unit may be used with monitoring units with different measuring capabilities. For example, the sensor unit may be used for a basic SpO₂ measurement (measurement mode 1) only, in which case only two emitter elements denoted with reference number 81 need to be active. The corresponding wavelengths may be, for example, 660 and 900 nm. Thus, in this case the activation status information may indicate that only measurement mode 1 is active, and emitter activation information is needed for input terminals 1 and 2 only. If a high precision SpO₂ measurement (measurement mode 2) is to be taken into use, four emitter elements, denoted with reference number 82 in the figure, need to be active. In this case the activation status information may indicate, after possible reconfiguration, that only measurement modes 1 and 2 are active. The necessary emitter activation information relates to drive input terminals 1 to 3 of the sensor unit and the corresponding wavelengths may be, for example, 660, 900, 632, and 720 nm. If the sensor is to be used with a monitoring unit capable of measuring fractional oxygen saturation (measurement mode 3), at least 6 emitter elements need to be employed, which are denoted with reference number 83 in the figure. In this case, the activation status information may indicate, after possible reconfiguration, that measurement modes 1 to 3 are active, and the sensor memory includes emitter activation information for at least drive input terminals 1 to 4. The at least 6 wavelengths may be employed to track the concentrations of HbO2, HbCO, and HbMet. If the sensor is used in operating theatres in connection with major surgeries, in which blood transfusions are likely to be needed, the monitoring unit may employ 8 to 10 emitter elements to be able to follow the concentrations of total hemoglobin and hematocrit (measurement modes 1 to 4 are active). After a certain number of use hours, the performance of the emitter elements 81 of the basic SpO₂ measurement is degraded so that the said elements have to be replaced by new emitter elements 84 having substantially the same wavelengths (measurement mode 5). Since the activation status information now reveals that the sensor unit is provided with an inactivated measurement mode with the same wavelength combination as measurement mode 1, measurement mode 1 may be inactivated and measurement mode 5 activated in the sensor ability update process (step 608). The degraded elements may also be inactivated by updating the emitter activation information, i.e. storing the new input terminal numbers for the said wavelengths. If the concentration of further blood substances, like glucose, is to be measured (measurement mode 6), all 16 emitter elements may be activated. In this state of the sensor unit, the activation status information then indicates that measurement modes 1-4 and 6, or measurement modes 2-6 are activated, depending on whether emitter elements 81 or 84 are used for the basic SpO₂ measurement.

As obvious from the above, the activation status information indicates the measurement modes available at present, even though the sensor is equipped with emitter elements for all possible measurement modes. The emitter activation information may be stored for the activated measurement modes only, or also for at least some of the measurement modes that are currently inactivated. In the former case, the emitter activation information is updated every time a new measurement mode is activated. The presence/absence of emitter activation information may thus serve as the activation status information.

The control unit may change the combination of wavelengths dynamically without user interaction. Depending on the blood parameters to be measured, this change of the wavelength combination may be carried out within one measurement mode or by dynamically changing the measurement mode over time. Thus, a certain measurement mode may be a combination of two or more other measurement modes or may include dynamic change of the wavelength combination as an intrinsic feature. Furthermore, it is even possible that the emitter activation information includes information for a greater number of wavelengths than the number of wavelengths currently available in the sensor, if the number of wavelengths (emitter elements) may be upgraded. However, in this case the information stored in the sensor may reveal that the sensor cannot be used with some of the wavelengths for which emitter activation information is stored. Based on the activation status information, the sensor type information and/or the general compatibility data the control unit may thus block the use of such extra wavelengths and inform the user of incompatibility issues relating to sensor usage. Generally, the sensor type information and the general compatibility data form a set of compatibility information based on which the monitoring unit may pre-check the compatibility of the sensor unit with any combination of wavelengths intended to be employed in the apparatus. Furthermore, based on the emitter activation information read from the sensor and the compatibility information stored elsewhere in the apparatus, the monitoring unit may guide the user to select a compatible sensor by displaying instructive messages, for example.

In a simple embodiment of the apparatus, the sensor-specific information may not include the sensor ability information, and the sensor ability maintenance unit may be configured to update the calibration data only. In some other embodiments of the apparatus, the activation status information may not be used at all, but the sensor ability information may include the service information only. In these embodiments, all emitter elements may be available all the time. However, the use of the activation status information enables longer use of the same sensor. In another embodiment, the sensor ability information (or the service information) may be in the form of information that indicates when a recalibration process is due, such as timer information. That is, in one embodiment of the apparatus, the recalibration may be initiated at regular intervals. It is also possible that the sensor-specific information is in the interface unit only, in which case the information may be updated by the control unit or by an external entity through access interface 224. The sensor-specific information may also be distributed between different memories of the apparatus.

To increase compatibility, a multiwavelength monitoring unit 230 may be made compatible with a standard two-wavelength sensor, since the pin order of terminal 250 may be such that the said standard sensor may be connected directly to connector 250. In this case, the interface unit is not needed.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.

Various aspects of the present invention are defined in the following numbered clauses:
1. A measuring apparatus for determining the amount of at least one substance in blood of a subject, the measuring apparatus comprising:
   - a sensor unit comprising
      - an emitter unit comprising a first plurality of emitter elements configured to emit radiation at a second plurality of wavelengths; and
      - a detector unit configured to receive radiation generated by the emitter unit and transmitted through tissue of a subject, wherein the detector unit is further configured to produce measurement signals indicative of absorption caused by blood of the subject;
   - a first memory storing sensor-specific information about the sensor unit, wherein the sensor-specific information includes at least calibration data for calibrating the apparatus for a selected measurement mode that corresponds to a given combination of wavelengths; and
   - a sensor ability maintenance unit configured to perform a sensor ability update process in which at least part of the sensor-specific information is updated, thereby to update ability of the sensor unit to operate in the selected measurement mode.
2. The measuring apparatus according to clause 1, wherein the sensor-specific information includes service information indicating whether the sensor ability update process needs to be initiated, in which the sensor ability update process is intended to improve performance of the sensor unit in the selected measurement mode.
3. The measuring apparatus according to clause 1 or clause 2, wherein the sensor-specific information further includes activation status information indicating whether the sensor ability update process needs to be initiated, in which the sensor ability update process is intended to activate resources of the sensor unit for the selected measurement mode.
4. The measuring apparatus according to any preceding clause, wherein the first memory is in the sensor unit.
5. The measuring apparatus according to any preceding clause, wherein the apparatus further comprises a user information display unit configured to indicate when the sensor ability update process needs to be performed.
6. The measuring apparatus according to any preceding clause, wherein the sensor ability maintenance unit is configured to update the calibration data and the service information in the sensor ability update process.
7. The measuring apparatus according to any preceding clause, wherein the sensor ability maintenance unit is configured to update the activation status information.
8. The measuring apparatus according to any preceding clause, wherein the sensor-specific information further comprises emitter activation data indicating how to switch on at least some of the first plurality of emitter elements, and wherein the sensor ability maintenance unit is configured to update the emitter activation data.
9. The measuring apparatus according to any preceding clause, wherein the sensor ability maintenance unit is further configured to collect history data concerning sensor usage and sensor-specific characteristics and update the service information based on the history data.
10. The measuring apparatus according to any preceding clause, wherein the sensor ability maintenance unit is configured to update the calibration data in the sensor ability update process.
11. The measuring apparatus according to any preceding clause, wherein the sensor ability maintenance unit is configured to update the service information at least at end of a measurement session.
12. A physiological sensor for use in determining the amount of at least one substance in blood of a subject, the physiological sensor being attachable to the subject and comprising:
   - an emitter unit comprising a first plurality of emitter elements configured to emit radiation at a second plurality of wavelengths;
   - a detector unit configured to receive radiation generated by the emitter unit and transmitted through tissue of the subject, wherein the detector unit is further configured to produce measurement signals indicative of absorption caused by blood of the subject;
   - a sensor memory storing sensor-specific information about the sensor unit, wherein the sensor-specific information includes at least calibration data for a given measurement mode; and
   - a memory access interface for enabling an entity external to the physiological sensor to update at least part of the sensor-specific information in a sensor ability update process, thereby to update ability of the sensor unit to operate in the given measurement mode.
13. The physiological sensor according to clause 12, wherein the sensor-specific information comprises sensor ability information indicating whether the sensor ability update process needs to be initiated.
14. The physiological sensor according to clause 13, wherein the sensor ability information includes service information indicating whether the sensor ability update process needs to be initiated, in which the sensor ability update process is intended to improve performance of the physiological sensor in the given measurement mode.
15. The physiological sensor according to clause 13, wherein the sensor ability information includes activation status information indicating whether the sensor ability update process needs to be initiated, in which the sensor ability update process is intended to activate resources of the physiological sensor for the given measurement mode.
16. The physiological sensor according to any of clauses 12 to 15, wherein sensor-specific information further comprises emitter activation data indicating how to switch on at least some of the first plurality of emitter elements.
17. The physiological sensor according to any of clauses 12 to 16, wherein the sensor-specific information further comprises history data concerning sensor usage and sensor-specific characteristics.
18. An interface unit for use in determining the amount of at least one substance in blood of a subject, the interface unit comprising:
   - a first interface for connecting the interface unit to a monitoring unit;
   - a second interface for connecting the interface unit to a sensor unit comprising a first plurality of emitter elements configured to emit radiation at a second plurality of wavelengths;
   - an emitter switching unit configured to connect drive current generated by the monitoring unit to the sensor unit through the second interface;
   - a memory storing sensor-specific information about the sensor unit, wherein the sensor-specific information includes at least calibration data for a given measurement mode; and
   - a memory access interface for enabling an entity external to the interface unit to update at least part of the sensor-specific information in a sensor ability update process, thereby to update ability of the sensor unit to operate in the given measurement mode.
19. The interface unit according to clause 18, wherein the sensor-specific information comprises sensor ability information indicating whether the sensor ability update process needs to be initiated.
20. The interface unit according to clause 18 or clause 19, wherein the memory stores sensor-specific information for a plurality of sensor units connectable to the interface unit.

## Claims

1. A measuring apparatus for determining the amount of at least one substance in blood of a subject, the measuring apparatus comprising:
- a sensor unit (210) comprising
- an emitter unit (211) comprising a first plurality of emitter elements configured to emit radiation at a second plurality of wavelengths; and
- a detector unit (214) configured to receive radiation generated by the emitter unit and transmitted through tissue of a subject, wherein the detector unit is further configured to produce measurement signals indicative of absorption caused by blood of the subject;
- a first memory (216; 222) storing sensor-specific information about the sensor unit, wherein the sensor-specific information includes at least calibration data for calibrating the apparatus for a selected measurement mode that corresponds to a given combination of wavelengths; and
- a sensor ability maintenance unit (231, 236; 237) configured to perform a sensor ability update process in which at least part of the sensor-specific information is updated, thereby to update the ability of the sensor unit (210) to operate in the selected measurement mode.

2. The measuring apparatus according to claim 1, wherein the sensor-specific information includes service information indicating whether the sensor ability update process needs to be initiated, in which the sensor ability update process is intended to improve performance of the sensor unit(210) in the selected measurement mode.

3. The measuring apparatus according to claim 1 or claim 2, wherein the sensor-specific information further includes activation status information indicating whether the sensor ability update process needs to be initiated, in which the sensor ability update process is intended to activate resources of the sensor unit (210) for the selected measurement mode.

4. The measuring apparatus according to any preceding claim, wherein the sensor ability maintenance unit (231, 236; 237) is configured to update the calibration data and the service information in the sensor ability update process.

5. The measuring apparatus according to claim 3 or 4, wherein the sensor ability maintenance unit (231, 236; 237) is configured to update the activation status information.

6. The measuring apparatus according to any preceding claim, wherein the sensor ability maintenance unit (231, 236; 237) is configured to update the calibration data in the sensor ability update process.

7. The measuring apparatus according to any preceding claim, wherein the sensor ability maintenance unit (231, 236; 237) is configured to update the service information at least at the end of a measurement session.

8. A physiological sensor for use in determining the amount of at least one substance in blood of a subject, the physiological sensor (210) being attachable to the subject and comprising the measuring apparatus of any one of the preceding claims and including
- a memory access interface (217) for enabling the sensor ability maintenance unit (231, 236; 237) external to the physiological sensor to update at least part of the sensor-specific information in the sensor ability update process, thereby to update the ability of the sensor unit to operate in the given measurement mode.

9. The physiological sensor according to claim 8, wherein sensor-specific information further includes emitter activation data indicating how to switch on at least some of the first plurality of emitter elements (213, 214).

10. The physiological sensor according to claim 8 or claim 9, wherein the sensor-specific information further includes history data concerning sensor usage and sensor-specific characteristics.

11. An interface unit for use in determining the amount of at least one substance in blood of a subject, the interface unit (220) comprising:
- a first interface (250) for connecting the interface unit to a monitoring unit (230);
- a second interface (240) for connecting the interface unit to a sensor unit (210) comprising a first plurality of emitter elements (213, 214) configured to emit radiation at a second plurality of wavelengths;
- an emitter switching unit (221) configured to connect drive current generated by the monitoring unit to the sensor unit through the second interface (240);
- a memory (222) storing sensor-specific information about the sensor unit, wherein the sensor-specific information includes at least calibration data for a given measurement mode; and
- a memory access interface (224) for enabling an entity (231, 236; 237) external to the interface unit to update at least part of the sensor-specific information in a sensor ability update process, thereby to update ability of the sensor unit (210) to operate in the given measurement mode.

12. The interface unit according to claim 11, wherein the sensor-specific information comprises sensor ability information indicating whether the sensor ability update process needs to be initiated.

13. The interface unit according to claim 11 or claim 12, wherein the memory (222) stores sensor-specific information for a plurality of sensor units (210) connectable to the interface unit.

## Patentansprüche

1. Messvorrichtung zum Bestimmen der Menge von zumindest einer Substanz im Blut einer Testperson, die Messvorrichtung umfassend:
- eine Sensoreinheit (210), umfassend:
- eine Emittereinheit (211), die eine erste Vielzahl von Emitterelementen umfasst, die zum Aussenden von Strahlung auf einer zweiten Vielzahl von Wellenlängen konfiguriert sind; und
- eine Detektoreinheit (214), die zum Empfangen von Strahlung, welche durch die Emittereinheit erzeugt und durch Gewebe einer Testperson übertragen wird, konfiguriert ist, wobei die Detektoreinheit ferner zum Erzeugen von Messsignalen konfiguriert ist, die Absorption anzeigen, welche durch Blut der Testperson bewirkt ist;
- einen ersten Speicher (216; 222), der sensorspezifische Information über die Sensoreinheit speichert, wobei die sensorspezifische Information zumindest Kalibrationsdaten zum Kalibrieren der Vorrichtung für einen ausgewählten Messmodus enthält, der einer gegebenen Kombination von Wellenlängen entspricht; und
- eine Sensorfähigkeitswartungseinheit (231, 236; 237), die zum Ausführen eines Sensorfähigkeitsaktualisierungsprozesses konfiguriert ist, in dem zumindest ein Teil der sensorspezifischen Information aktualisiert wird, um dadurch die Fähigkeit der Sensoreinheit (210) zum Arbeiten im ausgewählten Messmodus zu aktualisieren.

2. Messvorrichtung nach Anspruch 1, wobei die sensorspezifische Information Dienstinformation beinhaltet, die anzeigt, ob der Sensorfähigkeitsaktualisierungsprozess eingeleitet werden muss, wobei der Sensorfähigkeitsaktualisierungsprozess zum Verbessern der Leistung der Sensoreinheit (210) im ausgewählten Messmodus bestimmt ist.

3. Messvorrichtung nach einem der Ansprüche 1 oder 2, wobei die sensorspezifische Information ferner Aktivierungsstatusinformation beinhaltet, die anzeigt, ob der Sensorfähigkeitsaktualisierungsprozess eingeleitet werden muss, wobei der Sensorfähigkeitsaktualisierungsprozess zum Aktivieren von Ressourcen für die Sensoreinheit (210) für den ausgewählten Messmodus bestimmt ist.

4. Messvorrichtung nach einem der vorhergehenden Ansprüche, wobei die Sensorfähigkeitswartungseinheit (231, 236; 237) zum Aktualisieren der Kalibrationsdaten und der Dienstinformation im Sensorfähigkeitsaktualisierungsprozess konfiguriert ist.

5. Messvorrichtung nach einem der Ansprüche 3 oder 4, wobei die Sensorfähigkeitswartungseinheit (231, 236; 237) zum Aktualisieren der Aktivierungsstatusinformation im Sensorfähigkeitsaktualisierungsprozess konfiguriert ist.

6. Messvorrichtung nach einem der vorhergehenden Ansprüche, wobei die Sensorfähigkeitswartungseinheit (231, 236; 237) zum Aktualisieren der Kalibrationsdaten im Sensorfähigkeitsaktualisierungsprozess konfiguriert ist.

7. Messvorrichtung nach einem der vorhergehenden Ansprüche, wobei die Sensorfähigkeitswartungseinheit (231, 236; 237) zum Aktualisieren der Dienstinformation zumindest am Ende einer Messsitzung konfiguriert ist.

8. Physiologischer Sensor zum Gebrauch zum Bestimmen der Menge von zumindest einer Substanz im Blut einer Testperson, wobei der physiologische Sensor (210) an der Testperson anbringbar ist und die Messvorrichtung gemäß einem der vorhergehenden Ansprüche umfasst und Folgendes enthält:
- eine Speicherzugangsschnittstelle (217) zum Ermöglichen, dass die Sensorfähigkeitswartungseinheit (231, 236; 237), die extern zum physiologischen Sensor ist, zumindest einen Teil der sensorspezifischen Information im Sensorfähigkeitsaktualisierungsprozess aktualisiert, um dadurch die Fähigkeit der Sensoreinheit zum Arbeiten im gegebenen Messmodus zu aktualisieren.

9. Physiologischer Sensor nach Anspruch 8, wobei die sensorspezifische Information ferner Emitteraktivierungsdaten beinhaltet, die anzeigen, wie zumindest einige der ersten Vielzahl von Emitterelementen (213, 214) einzuschalten sind.

10. Physiologischer Sensor nach einem der Ansprüche 8 oder 9, wobei die sensorspezifische Information ferner Verlaufsdaten beinhaltet, die Sensorgebrauch und sensorspezifische Kennzeichen betreffen.

11. Schnittstelleneinheit zum Gebrauch beim Bestimmen der Menge von zumindest einer Substanz im Blut einer Testperson, die Schnittstelleneinheit (220) umfassend:
- eine erste Schnittstelle (250) zum Verbinden der Schnittstelleneinheit mit einer Überwachungseinheit (230);
- eine zweite Schnittstelleneinheit (240) zum Verbinden der Schnittstelleneinheit mit einer Sensoreinheit (210), die eine erste Vielzahl von Emitterelementen (213, 214) umfasst, welche zum Aussenden auf einer zweiten Vielzahl von Wellenlängen konfiguriert sind;
- eine Emitterschalteinheit (221), die zum Verbinden von Ansteuerungsstrom, der durch die Überwachungseinheit erzeugt ist, mit der Sensoreinheit durch die zweite Schnittstelle (240) konfiguriert ist;
- einen Speicher (222), der sensorspezifische Information über die Sensoreinheit speichert, wobei die sensorspezifische Information zumindest Kalibrationsdaten für einen gegebenen Messmodus beinhaltet; und
- eine Speicherzugriffsschnittstelle (224) zum Ermöglichen, dass eine Einheit (231, 236; 237), die extern zur Schnittstelleneinheit ist, zumindest einen Teil der sensorspezifischen Information in einem Sensorfähigkeitsaktualisierungsprozess aktualisiert, um dadurch die Fähigkeit der Sensoreinheit (210) zum Arbeiten im gegebenen Messmodus zu aktualisieren.

12. Schnittstelleneinheit nach Anspruch 11, wobei die sensorspezifische Information Sensorfähigkeitsinformation umfasst, die anzeigt, ob der Sensorfähigkeitsaktualisierungsprozess eingeleitet werden muss.

13. Schnittstelleneinheit nach einem der Ansprüche 11 oder 12, wobei der Speicher (222) sensorspezifische Information für mehrere Sensoreinheiten (210) speichert, die mit der Schnittstelleneinheit verbindbar sind.

## Revendications

1. Appareil de mesure pour déterminer la quantité d'au moins une substance dans le sang d'un sujet, l'appareil de mesure comprenant :
- une unité de capteur (210) comprenant :
- une unité d'émetteur (211) comprenant une première pluralité d'éléments émetteurs configurés pour émettre un rayonnement à une seconde pluralité de longueurs d'onde ; et
- une unité de détecteur (214) configurée pour recevoir un rayonnement généré par l'unité d'émetteur et transmis à travers le tissu d'un sujet, dans lequel l'unité de détecteur est encore configurée pour produire des signaux de mesure indicatifs de l'absorption provoquée par le sang du sujet ;
- une première mémoire (216 ; 222) stockant des informations spécifiques au capteur sur l'unité de capteur et dans lequel les informations spécifiques au capteur comprennent au moins des données d'étalonnage pour étalonner l'appareil pour un mode de mesure choisi qui correspond à une combinaison donnée de longueurs d'onde ; et
- une unité de maintien de la capacité du capteur (231, 236 ; 237) configurée pour effectuer un processus de mise à jour de la capacité du capteur, dans lequel au moins une partie des informations spécifiques au capteur sont mises à jour, en sorte de mettre à jour la capacité de l'unité de capteur (210) à opérer dans le mode de mesure choisi.

2. Appareil de mesure selon la revendication 1, dans lequel les informations spécifiques au capteur comprennent des informations de service indiquant si le processus de mise à jour de la capacité du capteur doit être initié, dans lequel le processus de mise à jour de la capacité du capteur est destiné à améliorer la performance de l'unité de capteur (210) dans le mode de mesure choisi.

3. Appareil de mesure selon la revendication 1 ou la revendication 2, dans lequel les informations spécifiques au capteur comprennent en outre des informations d'état d'activation indiquant si le processus de mise à jour de la capacité du capteur doit être initié, dans lequel le processus de mise à jour de la capacité du capteur est destiné à activer les ressources de l'unité de capteur (210) pour le mode de mesure choisi.

4. Appareil de mesure selon l'une quelconque des revendications précédentes, dans lequel l'unité de maintien de la capacité du capteur (231, 236 ; 237) est configurée pour mettre à jour les données d'étalonnage et les informations de service dans le processus de mise à jour de la capacité du capteur.

5. Appareil de mesure selon la revendication 3 ou la revendication 4, dans lequel l'unité de maintien de la capacité du capteur (231, 236 ; 237) est configurée pour mettre à jour les informations d'état d'activation.

6. Appareil de mesure selon l'une quelconque des revendications précédentes, dans lequel l'unité de maintien de la capacité du capteur (231, 236 ; 237) est configurée pour mettre à jour les données d'étalonnage dans le processus de mise à jour de la capacité du capteur.

7. Appareil de mesure selon l'une quelconque des revendications précédentes, dans lequel l'unité de maintien de la capacité du capteur (231, 236 ; 237) est configurée pour mettre à jour les informations de service au moins à la fin d'une séance de mesure.

8. Capteur physiologique pour utilisation dans la détermination de la quantité d'au moins une substance dans le sang d'un sujet, le capteur physiologique (210) pouvant être fixé au sujet et comprenant l'appareil de mesure selon l'une quelconque des revendications précédentes et comprenant :
- une interface d'accès à la mémoire (217) pour permettre à l'unité d'entretien de la capacité du capteur (231, 236 ; 237) extérieure au capteur physiologique de mettre à jour au moins une partie des informations spécifiques au capteur dans le processus de mise à jour de la capacité du capteur, en sorte de mettre à jour la capacité de l'unité de capteur à opérer dans le mode de mesure donné.

9. Capteur physiologique selon la revendication 8, dans lequel les informations spécifiques au capteur comprennent en outre des données d'activation de l'émetteur indiquant comment se commuter sur au moins certains de la première pluralité d'éléments émetteurs (213, 214).

10. Capteur physiologique selon la revendication 8 ou la revendication 9, dans lequel les informations spécifiques au capteur comprennent en outre des données historiques concernant l'usage du capteur et les caractéristiques spécifiques au capteur.

11. Unité d'interface pour utilisation dans la détermination de la quantité d'au moins une substance dans le sang d'un sujet, l'unité d'interface (220) comprenant :
- une première interface (250) pour raccorder l'unité d'interface à une unité de surveillance (230) ;
- une seconde interface (240) pour raccorder l'unité d'interface à une unité de capteur (210) comprenant une première pluralité d'éléments émetteurs (213, 214) configurés pour émettre un rayonnement à une seconde pluralité de longueurs d'onde ;
- une unité de commutation d'émetteur (221) configurée pour connecter le courant d'entraînement généré par l'unité de surveillance à l'unité de capteur via la seconde interface (240) ;
- une mémoire (222) stockant les informations spécifiques au capteur sur l'unité de capteur, dans lequel les informations spécifiques au capteur comprennent au moins des données d'étalonnage pour un mode de mesure donné ; et
- une interface d'accès à la mémoire (224) pour permettre à une entité (231, 236 ; 237) externe à l'unité d'interface de mettre à our au moins une partie des informations spécifiques au capteur dans un processus de mise à jour de la capacité du capteur, en sorte de mettre à jour la capacité de l'unité de capteur (210) à opérer dans le mode de mesure donné.

12. Unité d'interface selon la revendication 11, dans laquelle les informations spécifiques au capteur comprennent des informations de capacité du capteur indiquant si le processus de mise à jour de la capacité du capteur doit être initié.

13. Unité d'interface selon la revendication 11 ou la revendication 12, dans laquelle la mémoire (222) stocke des informations spécifiques au capteur pour une pluralité d'unités de capteur (210) qui peuvent être connectées à l'unité d'interface.
